# NEW EUROPEAN PATENT SPECIFICATION

(11) **EP 2 083 887 B2**
(45) Date of publication and mention of the opposition decision: **30.09.2015**
(45) Mention of the grant of the patent: 02.02.2011
(21) Application number: 07824561.0
(22) Date of filing: 13.11.2007
(51) Int. Cl.: A61M 5/20

(54) **INJECTION DEVICE**
INJEKTIONSVORRICHTUNG
DISPOSITIF D'INJECTION

(30) Priority: 13.11.2006 GB 0622570
(43) Date of publication of application: 05.08.2009
(73) Proprietor: Cilag GmbH International, 6300 Zug (CH)
(72) Inventor: BURNELL, Rosemary Louise, Cambridge CB1 7TS (GB); CORRIGAN, Joseph Peter, London W13 8JZ (GB); JENNINGS, Douglas Ivan, Royston, Hertordshire SG8 7XU (GB)
(74) Representative: Tunstall, Christopher Stephen
(86) International application number: PCT/GB2007/004335
(87) International publication number: WO 2008/059233

(56) References cited:
- EP-A2- 0 666 084
- WO-A-2005/115510
- WO-A-2006/106294
- GB-A- 2 424 835
- GB-A- 2 424 838
- US-A1- 2006 189 938
- US-B1- 6 575 939

## Description

### FIELD OF THE INVENTION

The present invention relates to an injection device of the type that receives a syringe, extends it, discharges its contents and then retracts it automatically.

### BACKGROUND OF THE INVENTION

Previously known injection devices are described in WO95/35126 and EP-A-0516473. Such devices tend to employ a trigger that, when a releasable locking mechanism is engaged, may be operated to cause a drive spring to act upon a syringe.

Generally, in such devices, the trigger is rotatable about a pivot axis so that when it is depressed at a first end, a second end (which normally engages the drive spring) is also rotated away from the drive spring, thereby releasing it, so that the syringe extends under the bias of the drive spring and its contents are discharged. The trigger comprises a protrusion that is engagable with a cut-out on the releasable locking mechanism when the releasable locking mechanism is engaged, thereby allowing the trigger to be activated. When the releasable locking mechanism is not engaged, the protrusion abuts a portion of the releasable locking mechanism and thus prevents rotation of the trigger and release of the drive spring. In this way, accidental activation of the trigger can be prevented.

A problem with an injection device of this type is that the protrusion on the trigger flexes when a force is applied to the trigger and the releasable locking mechanism is not engaged. A strong force applied to the trigger can cause enough flex in the protrusion that the end of the protrusion can engage the cut-out on the releasable locking mechanism, thereby allowing the trigger to be activated even when the releasable locking mechanism has not been engaged. A co-pending UK patent application, published as GB 2424835, addresses this problem by providing a protrusion on the releasable locking mechanism and a cut-out on the trigger. In this way, when a force is applied to the trigger when the releasable locking mechanism is not engaged, the trigger and its protrusion both flex in such a way that the protrusion is forced away from the cut-out, thereby decreasing the risk of accidental activation of the trigger.

The injection device described in GB 2424835 requires a particular sequence of operation to fire the device. Specifically, the releasable locking mechanism must be engaged before the trigger can be activated. If the trigger is first held down before the releasable locking mechanism is activated, the trigger is forced against the protrusion so that the releasable locking mechanism cannot be moved into is engaged position (i.e. its position in which activation of trigger is possible). Movement of the releasable locking mechanism is prevented in this way by the presence of a ridge on the trigger over which the protrusion cannot pass when the trigger is held down before the releasable locking mechanism is activated. The sequential mode of operation that this configuration necessitates may pose difficulties for some users of the device.

### SUMMARY OF THE INVENTION

The injection device of the present invention is designed to overcome this and other problems.

In view of the foregoing and in accordance with a first aspect of the invention, there is provided an injection device according to claim 1.

Thus, the device may be activated by a user pressing and holding down the trigger and subsequently engaging the releasable locking mechanism. Preferably, the releasable locking mechanism forms part of a sliding sleeve which protrudes, in its first position, from the exit aperture and which is moved to its second position by pressing the sliding sleeve against the skin of a user. The above sequence of operation greatly assists users of the device who may find it difficult to hold the injection device against their skin before pressing down on the trigger. This arrangement may additionally allow the device to be operated by a user engaging the releasable locking mechanism and subsequently pressing the trigger.

In addition, the locking component may extend from a first end of the trigger in a direction along the first axis and includes a cut-out therein and the locking element comprises a protrusion along a second axis for communicating with a contact surface of the locking component when the releasable locking mechanism is in its first position and for communicating with the cut-out when the releasable locking mechanism is in its second position, wherein the second axis is at an angle to the first axis.

The cammed surface may be angled in such a way that the application of pressure to the activation surface of the trigger, when the releasable locking mechanism is in its first position, biases the protrusion of the locking element away from the cut-out of the locking component. Preferably, the cammed surface is sloped away from the protrusion in a direction away from the first end of the trigger.

Additionally, the protrusion may comprise a ridge adapted to communicate with an edge of the cut-out when the releasable locking mechanism is in its second position and the trigger is in its active position, thereby preventing movement of the trigger from its active position to its rest position. Thus, the trigger can be maintained in a rotated position following its activation, thereby serving to indicate that the injection device has been used.

Furthermore, the locking component may further include a first portion which extends into the cut-out from the locking component and which is arranged to communicate with the ridge when the releasable locking mechanism is in its second position and the trigger is in its active position.

Preferably, the releasable locking mechanism comprises biasing means arranged to bias the protrusion against the first portion of the locking component. Advantageously, the protrusion may comprise a sloped surface, which is angled with respect to the second axis. This feature ensures that the protrusion enters the cut-out smoothly when the releasable locking mechanism is moved to its second position.

Preferably, the first axis and the second axis are perpendicular to each other, thereby ensuring that the protrusion and the locking component are optimally arranged to ensure that the protrusion enters the cut-out when the releasable locking mechanism is moved to its second position, but also means that the protrusion and the locking component will flex in such a way as to avoid accidental activation of the trigger when the releasable locking mechanism is in its first position.

Preferably, the releasable locking mechanism comprises a sleeve, which protrudes from the exit aperture when the releasable locking mechanism is in its first position. Even more preferably, the releasable locking mechanism is biased into its first position when it is not activated.

### BRIEF DESCRIPTION OF THE DRAWINGS

The invention will now be described by way of example with reference to the accompanying drawings, in which:
Figure 1 shows a perspective view of an injection device according to the present invention;
Figure 2 shows a side view of the injection device of figure 1 with an upper section of its housing not shown;
Figure 3 shows a side view of the injection device of figure 2 with further components not shown;
Figure 4 shows a top plan view of the injection device of figure 3;
Figure 5 shows a perspective view of a trigger and releasable locking mechanism according to an embodiment of the present invention;
Figure 6 shows an alternative perspective view of the trigger and releasable locking mechanism of figure 5; and
Figure 7 shows a side cross-sectional view of the trigger and releasable locking mechanism of figures 5 and 6.

### DETAILED DESCRIPTION OF THE DRAWINGS

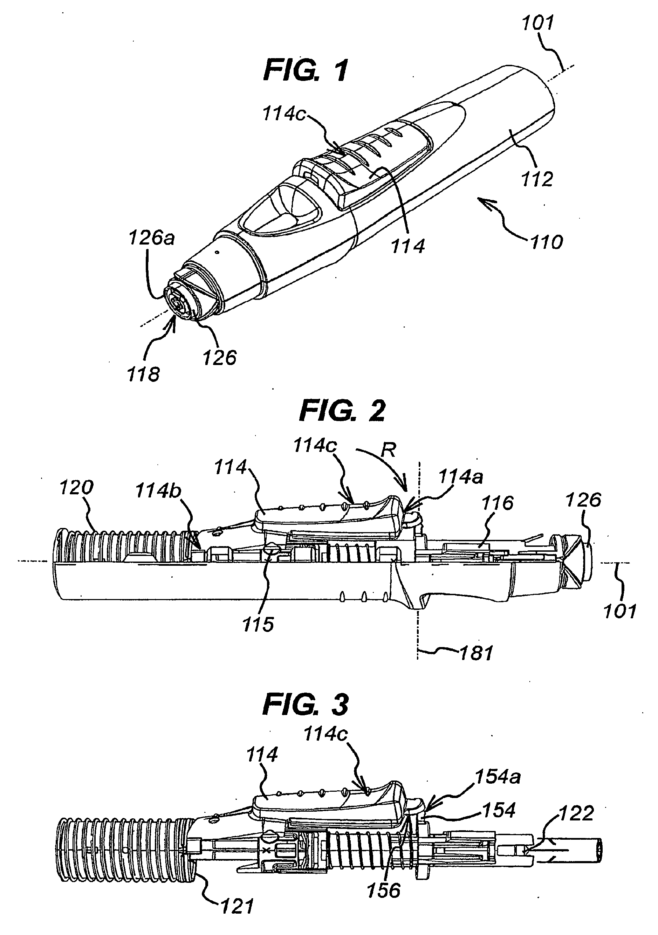

Figures 1 to 4 show an injection device (110) and its internal components according to one embodiment of the present invention. The injection device (110) has an injection device housing (112) and a longitudinal axis (101).

A syringe (122) is contained in the housing (112). The injection device (110) comprises a trigger (114) and a releasable locking mechanism (116). The trigger (114) has a first end (114a) and a second end (114b). The trigger (114) is rotatable about a pivot (115) from a rest position (as shown in figure 2) to an active position (not shown) by applying downwards pressure in direction R (into the injection device (110)) onto an activation surface (114c). The second end (114b) of the trigger (114) connects with a drive coupling (121) which is acted upon by a drive spring (120). The drive coupling (121) is in communication with the syringe (122).

Rotation of the trigger (114) about the pivot (115) near direction R (i.e. downwards into the housing (112) at its first end (114a)) causes the second end (114b) of the trigger (114) to disengage from the drive coupling (121) thereby letting the drive spring (120) drive the syringe (122) (via the drive coupling (121)) along the longitudinal axis (101) out of an aperture (118) in the housing (112).

The releasable locking mechanism (116) is in communication with a sliding sleeve (126) which protrudes, when in a first position, from the aperture (118) in the housing (112). The releasable locking mechanism (116) is activated by movement of the sliding sleeve (126) along the longitudinal axis (101) into the housing (112) into a second position.

A first end (126a) of the sliding sleeve (126) can be placed against the body into which a drug is being delivered, thereby deactivating the releasable locking mechanism (116) and allowing the trigger (114) to rotate in direction R from its rest position to its active position.

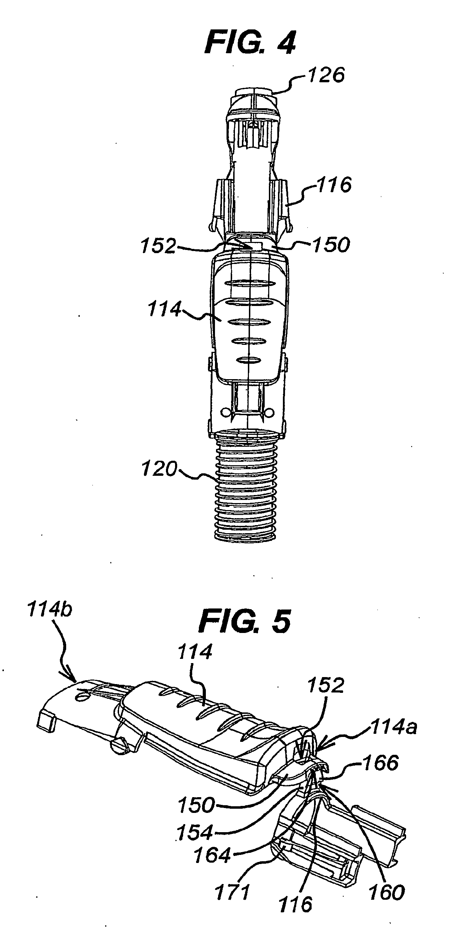

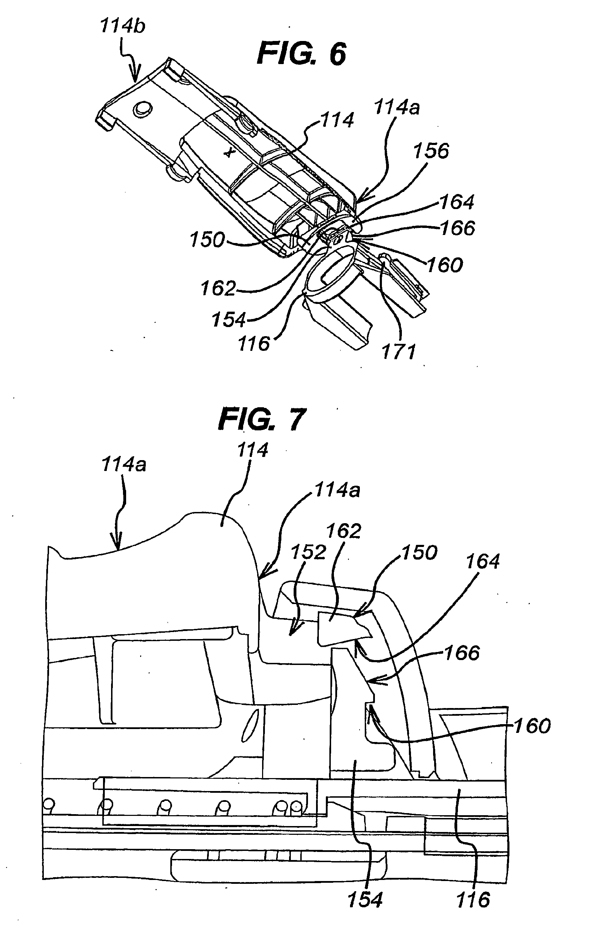

As can be seen from figures 5 and 6, the trigger (114) is provided at its first end (114a) with a locking component (150) having a cut-out (152). The locking component (150) extends from the first end (114a) of the trigger (114) in a direction substantially parallel to the longitudinal axis (101).

The releasable locking mechanism (116) includes a locking element (154) that takes the form of a protrusion (154), which projects in a direction along a perpendicular axis (181) that is perpendicular to the longitudinal axis (101). The cut-out (152) is dimensioned to receive the protrusion (154).

When the releasable locking mechanism (116) is in its first position, as shown in figures 2 to 6, an end (154a) of the protrusion (154) abuts an undersurface (156) of the locking component (150), thereby preventing rotation of the trigger (114).

When the releasable locking mechanism (116) is in its second position (not shown) following movement of the sliding sleeve (126) into the housing (112), the cut-out (152) is positioned above the end (154a) of the protrusion (154), allowing it to pass over the protrusion (154) while a downwards force is applied to the trigger (114). Hence, the trigger (114) is no longer prevented from rotating and disengages itself from the drive coupling (121), thereby extending the syringe (122).

The protrusion (154) comprises a ridge (166). The trigger (114) includes a first portion (162) that extends into the cut-out (152) from the locking component (150) of the trigger (114) and that is arranged to communicate with the first portion (162) following rotation of the trigger (114) so that the ridge (160) is locked over the first portion (162), thereby preventing movement of the trigger (114) from its active position back to its rest position.

The releasable locking mechanism (116) includes biasing means, in the form of resilient arms (171), which act against the internal surface of the housing (112) to bias the releasable locking mechanism (116) and sliding sleeve (126) in a direction out of the aperture (118). This way, following activation of the trigger (114), the ridge (160) is locked over the first portion (162) of the trigger (114), thereby holding the trigger (114) in its active position.

As will be seen from Figure 7, the locking component (150) of the trigger (114) comprises a cammed surface (164) on the under-surface (156) of the locking component (150) that is positioned between the cut-out (152) and the end of the locking component (150). The cammed surface (164) is sloped away from the protrusion (154) in the direction away from the first end (114a) of the trigger (114). The cammed surface (164) abuts the protrusion (154) when force is applied to the trigger (114) in a direction R and the releasable locking mechanism (116) is in its first position. This has the effect that when pressure is applied to the trigger (114), when the releasable locking mechanism (116) is in its first position, the locking component (150) and protrusion (154) flex so that the end (154a) of the protrusion is directed away from the cut-out (152). This prevents the trigger (114) (and hence injection device (110)) being accidentally operated by pushing down hard on the trigger (114) when the releasable locking mechanism is in its first position.

The cammed surface (164) is sloped such that the releasable locking mechanism (116) can be moved from its first position to its second position whilst pressure is being applied to the trigger (114), in such a way that the end (154a) of the protrusion (154) moves over the cammed surface (164) to the cut-out (152), thus allowing the trigger (114) to move from its rest position to its active position, thereby activating the injection device (110).

The protrusion (154) has a sloped surface (166) that is angled with respect to the perpendicular axis (181), which allows the first portion (162) of the trigger (114) to pass over the protrusion (154) more effectively when the trigger (114) moves from its rest position to its active position when the releasable locking mechanism (116) is in its second position.

It will of course be understood that the present invention has been described above purely by way of example and modifications of detail can be made within the scope of the invention.

## Claims

1. An injection device (110) comprising:
a housing (112) defining a first axis (101) and adapted to receive a syringe (122) having a discharge nozzle, so that the syringe (122) is movable between a retracted position, in which the discharge nozzle is contained within the housing (112), and an extended position, in which the discharge nozzle extends from the housing (112) through an exit aperture (118);
a drive (120) that is acted upon and in turn acts upon the syringe (122);
a trigger (114) movable from a rest position, in which it causes the drive (120) to be retained, to an active position, in which it no longer causes the drive (120) to be retained; and
a releasable locking mechanism (116) movable from a first position, in which the trigger (114) is prevented from moving into its active position, to a second position, in which the trigger (114) can be moved to its active position,
wherein the trigger (114) has an activation surface (114c) on which pressure can be applied to move the trigger from its rest position to its active position when the releasable locking mechanism (116) is in its second position,
wherein the trigger (114) and the releasable locking mechanism (116) are arranged such that the trigger (114) moves to its active position when the releasable locking mechanism (116) is moved to its second position whilst pressure is being applied to the activation surface (114c) of the trigger (114),
wherein the trigger (114) includes a locking component (150) and the releasable locking mechanism (116) includes a locking element (154) and wherein the locking component (150) and the locking element (154) are in contacting juxtaposition when the trigger (114) is in its rest position and the releasable locking mechanism (116) is in its first position, such that the trigger (114) is prevented from moving to its active position when pressure is applied to the activation surface (114c) of the trigger (114), and
**characterised in that** the locking component (150) comprises a cammed surface (164) on which the locking element (154) acts when the releasable locking mechanism (116) is in its first position, such that, whilst pressure is being applied to the activation surface (114c) of the trigger (114) in a direction substantially along a second axis into the injection device (110), the locking element (154) can be moved over the cammed surface (164), as the releasable locking mechanism (116) is moved to its second position, into a position in which the locking component (150) and the locking element (154) are not in contacting juxtaposition.

2. The injection device (110) of claim 1, wherein the locking component (150) and the locking element (154) are adapted such that, whilst pressure is being applied to the activation surface (114c) of the trigger (114) when the trigger (114) is initially in its rest position and the releasable locking mechanism (116) is initially in its first position, the releasable locking mechanism (116) can be moved to its second position in which the locking component (150) and the locking element (154) are not in contacting juxtaposition, such that the trigger (114) moves from its rest position to its active position.

3. The injection device (110) of claim 1 or claim 2, wherein the locking component (150) extends from a first end of the trigger (114) in a direction along the first axis (101) and includes a cut-out (152) therein and the locking element (154) comprises a protrusion along a second axis for communicating with a contact surface of the locking component (150) when the releasable locking mechanism (116) is in its first position and for communicating with the cut-out (152) when the releasable locking mechanism (116) is in its second position, wherein the second axis is at an angle to the first axis (101).

4. The injection device (110) of any one of the preceding claims, wherein the cammed surface (164) is angled in such a way that application of pressure to the activation surface (114c) of the trigger (114), when the releasable locking mechanism (116) is in its first position, biases the protrusion of the locking element (154) away from the cut-out (152) of the locking component (150).

5. The injection device (110) of any of the preceding claims, wherein the protrusion (154) comprises a ridge (160) adapted to communicate with an edge of the cut-out (152) when the releasable locking mechanism (116) is in its second position and the trigger (114) is in its active position, thereby preventing movement of the trigger (114) from its active position to its rest position.

6. The injection device (110) of claim 5, wherein the locking component (150) further includes a first portion (162) which extends into the cut-out (152) from the locking component (150) and which is arranged to communicate with the ridge (160) when the releasable locking mechanism (116) is in its second position and the trigger (114) is in its active position.

7. The injection device (110) of claim 6, wherein the releasable locking mechanism (116) comprises biasing means (171) arranged to bias the protrusion (154) against the first portion (162) of the locking component (150).

8. The injection device (110) of any of the preceding claims, wherein the protrusion (154) comprises a sloped surface (166), which is angled with respect to the second axis.

9. The injection device (110) of any of claims 3 to 8, wherein the first axis (101) and the second axis (181) are perpendicular to each other.

10. The injection device (110) of any preceding claim, wherein the trigger (114) is pivotally mounted on the housing (112).

11. The injection device (110) of any preceding claim, wherein the releasable locking mechanism (116) comprises a sleeve (126), which protrudes from the exit aperture (118) when the releasable locking mechanism (116) is in its first position.

12. The injection device (110) of any preceding claim, wherein the releasable locking mechanism (116) is biased into its first position when it is not activated.

## Patentansprüche

1. Injektionsvorrichtung (110), umfassend:
ein Gehäuse (112), das eine erste Achse (101) definiert und dazu bestimmt ist, eine Spritze (122) aufzunehmen, die eine Abgabedüse aufweist, wobei die Spritze (122) zwischen einer zurückgezogenen Position, in welcher die Abgabedüse innerhalb des Gehäuses (112) enthalten ist, und einer ausgefahrenen Position bewegbar ist, in welcher sich die Abgabedüse aus dem Gehäuse (112) durch eine Austrittsöffnung (118) herauserstreckt;
einen Antrieb (120), auf den eingewirkt wird und der seinerseits auf die Spritze (122) einwirkt;
einen Auslöser (114), der aus einer Ruheposition, in welcher er das Zurückhalten des Antriebes (120) bewirkt, in eine aktive Position bewegbar ist, in welcher er das Zurückhalten des Antriebes (120) nicht länger bewirkt; und
einen freigebbaren Verriegelungsmechanismus (116), der aus einer ersten Position, in welcher der Auslöser (114) daran gehindert wird, sich in seine aktive Position zu bewegen, in eine zweite Position bewegbar ist, in welcher der Auslöser (114) in seine aktive Position bewegt werden kann, wobei der Auslöser (114) eine Betätigungsfläche (114c) aufweist, auf welche Druck aufgebracht werden kann, um den Auslöser aus seiner Ruheposition in seine aktive Position zu bewegen, wenn sich der freigebbare Verriegelungsmechanismus (116) in seiner zweiten Position befindet,
wobei der Auslöser (114) und der freigebbare Verriegelungsmechanismus (116) derart angeordnet sind, dass sich der Auslöser (114) in seine aktive Position bewegt, wenn der freigebbare Verriegelungsmechanismus (116) in seine zweite Position bewegt wird, während Druck auf die Betätigungsfläche (114c) des Auslösers (114) aufgebracht wird,
wobei der Auslöser (114) eine Verriegelungskomponente (150) aufweist, und der freigebbare Verriegelungsmechanismus (116) ein Verriegelungselement (154) aufweist, und wobei sich die Verriegelungskomponente (150) und das Verriegelungselement (154) in einander gegenüberliegender kontaktierender Position befinden, während sich der Auslöser (114) in seiner Ruheposition und der freigebbare Verriegelungsmechanismus (116) sich in seiner ersten Position befindet, derart, dass der Auslöser (114) daran gehindert wird, sich in seine aktive Position zu bewegen, wenn Druck auf die Betätigungsfläche (114c) des Auslösers (114) aufgebracht wird,
**dadurch gekennzeichnet, dass** die Verriegelungskomponente (150) eine Nockenfläche (164) aufweist, auf welche das Verriegelungselement (154) einwirkt, wenn sich der freigebbare Verriegelungsmechanismus (116) in seiner ersten Position befindet, derart dass,
während Druck auf die Betätigungsfläche (114c) des Auslösers (114) in einer Richtung im Wesentlichen entlang der zweiten Achse in die Injektionsvorrichtung (110) aufgebracht wird, das Verriegelungselement (154) über die Nockenfläche (164) bewegt werden kann, wenn der freigebbare Verriegelungsmechanismus (116) in seine zweite Position bewegt wird, in eine Position, in welcher sich die Verriegelungskomponente (150) und das Verriegelungselement (154) nicht in einander gegenüberliegender kontaktierender Position befinden.

2. Injektionsvorrichtung (110) nach Anspruch 1, bei welcher die Verriegelungskomponente (150) und das Verriegelungselement (154) so ausgebildet sind, dass, während Druck auf die Betätigungsfläche (114c) des Auslösers (114) aufgebracht wird, wenn sich der Auslöser (114) anfänglich in seiner Ruheposition und der freigebbare Verriegelungsmechanismus (116) sich anfänglich in seiner ersten Position befindet, der freigebbare Verriegelungsmechanismus (116) in seine zweite Position bewegt werden kann, in welcher sich die Verriegelungskomponente (150) und das Verriegelungselement (154) nicht in einander gegenüberliegender kontaktierender Position befinden, derart, dass sich der Auslöser (114) aus seiner Ruheposition in seine aktive Position bewegt.

3. Injektionsvorrichtung (110) nach Anspruch 1 oder 2, bei welcher sich die Verriegelungskomponente (150) von einem ersten Ende des Auslösers (114) in einer Richtung entlang der ersten Achse (101) erstreckt und einen Ausschnitt (152) aufweist, und das Verriegelungselement (154) einen Vorsprung entlang einer zweiten Achse zur Kommunikation mit einer Kontaktfläche der Verriegelungskomponente (150) aufweist, wenn sich der freigebbare Verriegelungsmechanismus (116) in seiner ersten Position befindet, und zur Kommunikation mit dem Ausschnitt (152), wenn sich der freigebbare Verriegelungsmechanismus (116) in seiner zweiten Position befindet, wobei sich die zweite Achse unter einem Winkel zur ersten Achse (101) erstreckt.

4. Injektionsvorrichtung (110) nach einem der vorhergehenden Ansprüche, bei welcher die Nockenfläche (164) derart abgewinkelt ist, dass das Aufbringen von Druck auf die Betätigungsfläche (114c) des Auslösers (114), wenn sich der freigebbare Verriegelungsmechanismus (116) in seiner ersten Position befindet, den Vorsprung des Verriegelungselementes (154) von dem Ausschnitt (152) der Verriegelungskomponente (150) weg vorspannt.

5. Injektionsvorrichtung (110) nach einem der vorhergehenden Ansprüche, bei welcher der Vorsprung (154) eine Rippe (160) aufweist, die befähigt ist, mit einer Kante des Ausschnittes (152) zusammenzuwirken, wenn sich der freigebbare Verriegelungsmechanismus (116) in seiner zweiten Position und der Auslöser (114) sich in seiner aktiven Position befindet, wodurch eine Bewegung des Auslösers (114) von seiner aktiven Position in seine Ruheposition verhindert wird.

6. Injektionsvorrichtung (110) nach Anspruch 5, bei welcher die Verriegelungskomponente (150) ferner einen ersten Teil (162) aufweist, der sich von der Verriegelungskomponente (150) in den Ausschnitt (152) erstreckt und der so ausgebildet ist, dass er mit der Rippe (160) zusammenwirkt, wenn sich der freigebbare Verriegelungsmechanismus (116) in seiner zweiten Position und der Auslöser (114) sich in seiner aktiven Position befindet.

7. Injektionsvorrichtung (110) nach Anspruch 6, bei welcher der freigebbare Verriegelungsmechanismus (116) ein Vorspannmittel (171) aufweist, das so ausgebildet ist, dass es den Vorsprung (154) gegen den ersten Teil (162) der Verriegelungskomponente (150) vorspannt.

8. Injektionsvorrichtung (110) nach einem der vorhergehenden Ansprüche, bei welcher der Vorsprung (154) eine Schrägfläche (166) aufweist, die bezüglich der zweiten Achse abgewinkelt ist.

9. Injektionsvorrichtung (110) nach einem der Ansprüche 3 bis 8, bei welcher die erste Achse (101) und die zweite Achse (181) zueinander senkrecht stehen.

10. Injektionsvorrichtung (110) nach einem der vorhergehenden Ansprüche, bei welcher der Auslöser (114) an dem Gehäuse (112) schwenkbar montiert ist.

11. Injektionsvorrichtung (110) nach einem der vorhergehenden Ansprüche, bei welcher der freigebbare Verriegelungsmechanismus (116) eine Hülse (126) aufweist, die von der Austrittsöffnung (118) herausragt, wenn sich der freigebbare Verriegelungsmechanismus (116) in seiner ersten Position befindet.

12. Injektionsvorrichtung (110) nach einem der vorhergehenden Ansprüche, bei welcher der freigebbare Verriegelungsmechanismus (116) in seine erste Position vorgespannt ist, wenn er nicht betätigt ist.

## Revendications

1. Dispositif d'injection (110) comprenant :
un boîtier (112) définissant un premier axe (101) et conçu pour recevoir une seringue (122) présentant une buse d'évacuation, de sorte que la seringue (122) est mobile entre une position rentrée dans laquelle la buse d'évacuation est contenue à l'intérieur du boîtier (112) et une position sortie dans laquelle la buse d'évacuation s'étend depuis le boîtier (112) à travers une ouverture de sortie (118) ;
un entraînement (120) sur lequel une action est exercée et agissant à son tour sur la seringue (122),
une gâchette (114) mobile d'une position de repos dans laquelle elle amène l'entraînement (120) à être retenu à une position active dans laquelle elle n'amène plus l'entraînement (120) à être retenu ; et
un mécanisme de verrouillage libérable (116) mobile d'une position dans laquelle la gâchette (114) est empêchée de se déplacer dans sa position active à une seconde position dans laquelle la gâchette (114) peut être déplacée dans sa position active,
la gâchette (114) présentant une surface d'activation (114c) sur laquelle une pression peut être appliquée pour déplacer la gâchette (114) de sa position de repos à sa position active lorsque le mécanisme de verrouillage libérable (116) se trouve dans sa seconde position,
la gâchette (114) et le mécanisme de verrouillage libérable (116) étant disposés de telle sorte que la gâchette (114) se déplace dans sa position active lorsque le mécanisme de verrouillage libérable (116) est déplacé vers sa seconde position tandis qu'une pression est appliquée sur la surface d'activation (114c) de la gâchette (114),
la gâchette (114) comportant un composant de verrouillage (150) et le mécanisme de verrouillage libérable (116) comportant un élément de verrouillage (154) et le composant de verrouillage (150) et l'élément de verrouillage (154) étant en contact par juxtaposition lorsque la gâchette (114) se trouve dans sa position de repos et le mécanisme de verrouillage libérable (116) se trouve dans sa première position, de telle sorte que la gâchette (114) soit empêchée de se déplacer vers sa position active lorsqu'une pression est appliquée sur la surface d'activation (114c) de la gâchette (114), et
**caractérisé en ce que** le composant de verrouillage (150) comprend une surface à came (164) sur laquelle l'élément de verrouillage (154) agit lorsque le mécanisme de verrouillage libérable (116) se trouve dans sa première position, de telle sorte que, tandis qu'une pression est appliquée sur la surface d'activation (114c) de la gâchette (114) dans une direction sensiblement le long du second axe à l'intérieur du dispositif d'injection (110), l'élément de verrouillage (154) peut être déplacé au-dessus de la surface à came (164), à mesure que le mécanisme de verrouillage libérable (116) est déplacé vers sa seconde position, dans une position dans laquelle le composant de verrouillage (150) et l'élément de verrouillage (154) ne sont pas en contact par juxtaposition.

2. Dispositif d'injection (110) selon la revendication 1, le composant de verrouillage (150) et l'élément de verrouillage (154) étant conçus de telle sorte que, tandis qu'une pression est appliquée sur la surface d'activation (114c) de la gâchette (114) lorsque la gâchette (114) se trouve initialement dans sa position de repos et que le mécanisme de verrouillage libérable (116) se trouve initialement dans sa première position, le mécanisme de verrouillage libérable (116) peut être déplacé dans sa seconde position dans laquelle le composant de verrouillage (150) et l'élément de verrouillage (154) ne sont pas en contact par juxtaposition, de telle sorte que la gâchette (114) se déplace de sa position de repos à sa position active.

3. Dispositif d'injection (110) selon la revendication 1 ou la revendication 2, le composant de verrouillage (150) s'étendant d'une première extrémité de la gâchette (114) dans une direction le long du premier axe (101) et comportant une découpe (152) formée dedans et l'élément de verrouillage (154) comprenant une saillie le long d'un second axe pour communiquer avec une surface de contact du composant de verrouillage (150) lorsque le mécanisme de verrouillage libérable (116) se trouve dans sa première position et pour communiquer avec la découpe (152) lorsque le mécanisme de verrouillage libérable (116) se trouve dans sa seconde position, le second axe se trouvant sous un angle par rapport au premier axe (101).

4. Dispositif d'injection (110) selon l'une quelconque des revendications précédentes, la surface à came (164) étant inclinée de telle sorte que l'application d'une pression sur la surface d'activation (114c) de la gâchette (114), lorsque le mécanisme de verrouillage libérable (116) se trouve dans sa première position, sollicite la saillie de l'élément de verrouillage (154) à distance de la découpe (152) du composant de verrouillage (150).

5. Dispositif d'injection (110) selon l'une quelconque des revendications précédentes, la saillie (154) comprenant une nervure (160) conçue pour communiquer avec un bord de la découpe (152) lorsque le mécanisme de verrouillage libérable (116) se trouve dans sa seconde position et que la gâchette (114) se trouve dans sa position active, ce qui empêche un déplacement de la gâchette (114) de sa position active à sa position de repos.

6. Dispositif d'injection (110) selon la revendication 5, le composant de verrouillage (150) comportant en outre une première partie (162) s'étendant à l'intérieur de la découpe (152) depuis le composant de verrouillage (150) et conçue pour communiquer avec la nervure (160) lorsque le mécanisme de verrouillage libérable (116) se trouve dans sa seconde position et que la gâchette (114) se trouve dans sa position active.

7. Dispositif d'injection (110) selon la revendication 6, le mécanisme de verrouillage libérable (116) comprenant des moyens de sollicitation (171) disposés pour solliciter la saillie (154) contre la première partie (162) du composant de verrouillage (150).

8. Dispositif d'injection (110) selon l'une quelconque des revendications précédentes, la saillie (154) comprenant une surface en pente (166) inclinée par rapport au second axe.

9. Dispositif d'injection (110) selon l'une quelconque des revendications 3 à 8, le premier axe (101) et le second axe (181) étant perpendiculaires l'un à l'autre.

10. Dispositif d'injection (110) selon l'une quelconque des revendications précédentes, la gâchette (114) étant montée pivotante sur le boîtier (112).

11. Dispositif d'injection (110) selon l'une quelconque des revendications précédentes, le mécanisme de verrouillage libérable (116) comprenant un manchon (126) faisant saillie de l'ouverture de sortie (118) lorsque le mécanisme de verrouillage libérable se trouve dans sa première position.

12. Dispositif d'injection (110) selon l'une quelconque des revendications précédentes, le mécanisme de verrouillage libérable (116) étant sollicité pour entrer dans sa première position lorsqu'il n'est pas activé.
